# EUROPEAN PATENT APPLICATION

(11) **EP 3 326 573 A1**
(43) Date of publication of application: **30.05.2018**
(21) Application number: 16200803.1
(22) Date of filing: 25.11.2016
(51) Int. Cl.: A61C 9/00, A61B 8/12, A61B 8/00

(54) **INTRAORAL INTERFACE STRUCTURE FOR AN INTRAORAL SCANNING DEVICE**

(71) Applicant: RWTH Aachen University, 52062 Aachen (DE)
(72) Inventor: VOLLBORN, Thorsten, 52066 Aachen (DE); CHUEMBOU PEKAM, Fabrice, 52068 Aachen (DE); HABOR, Daniel, 52066 Aachen (DE); RADERMACHER, Klaus, 52222 Stolberg (DE); HEGER, Stefan, 69198 Schriesheim (DE)
(74) Representative: Eisenführ Speiser

(57) **Abstract**

The invention relates to an intraoral interface structure (100) for arrangement between an ultrasound transducer (36) of an intraoral scanning device (1) and a tooth region (90) or residual tooth region to be scanned, and for transmitting ultrasound waves from the transducer (36) to the tooth region (90) or residual tooth region, the intraoral interface structure (100) comprising: a frame member (102) defining an interior space (104) therein, the frame member (102) being adapted to be placed over the tooth region (90) or residual tooth region, such that the tooth region (90) or residual tooth region is at least partially received in said interior space (104), wherein the interior space (104) is adapted to be filled with a coupling medium (164) for impedance matching, the frame member (102) comprising an acoustic window (108) and a rim (106) around the acoustic window (108) for forming a receiving portion for a section of an intraoral scanning device (1), and first and second leg portions (110, 112) extending from said rim (106) for contacting opposing sides of the tooth region (90) or residual tooth region or tissue or bone adjacent to it such that the intraoral scanning device (1) is able to be supported thereat.

## Description

The invention relates to an intraoral interface structure for arrangement between an ultrasound transducer of an intraoral scanning device and a tooth region or residual tooth region to be scanned, and for transmitting ultrasound waves from the transducer to the tooth region or residual tooth region.

Moreover, the invention relates to a system, comprising an intraoral scanning device for according ultrasound scan data in the mouth of the patient and an intraoral interface structure of the aforementioned type. Moreover, the invention relates to a method for scanning a tooth region or residual tooth region.

One of the main steps of the design process of dental prosthetics such as crowns, bridges, etc., consists of taking an impression of the prepared dental region. The conventional method is to perform it manually using elastomer casting compounds. Despite the fact that it is time-consuming and error-prone, it is still the present gold standard. Indeed, blood, saliva, air bubbles or other particles can displace the elastomer material and thus decrease the quality of the impression significantly.

Taking an impression of the prepared dental region requires drying the area around the prepared dental region, so that saliva and other body fluids do not distort the impressioning result. When the preparation is deep, it might be necessary to remove gingiva by means of retraction cords or even by means of electrotome. This is painful and might cause serious problems.

When a silicon impression is used, this one is used as a mold for a plaster model, on which the crowns, bridges etc. are prepared in a dental laboratory. Also in these manufacturing steps failures may occur, which distort the accuracy of the fit of the prosthesis.

Both the need for long-time reliable restorations in the posterior region and the increased demand for aesthetic dental replacements in the anterior region has led to the development of aluminum and zirconia oxide ceramics which are nowadays used in dental restoration. These ceramics are difficult to manufacture and require the use of CAD/CAM technologies. It is an ongoing process to eliminate failure sources in the production process and to facilitate chairside manufacturing of the restoration, which reduces time and costs and provides comfort for the patient.

In this regard methods are used which use optical measuring technology to sample the surfaces of the prepared tooth region and which scan the mouth of the patient to provide a 3D data model which can be used in CAD/CAM production processes. These methods can be divided into two different approaches: In one approach, a silicon or plaster model is produced, which is then extraorally scanned. Alternatively, also intraoral scanners have been developed which do not require the separate impression taking via a silicon model.

Even though a plurality of these systems has been developed in the past, there is still the need to provide improved systems which further reduce failure sources and are more easy to use by the dentist. In particular, it is required to also record subgingivally located portions of the tooth, which is in particular difficult when covered with blood or saliva.

From EP 2 023 849 a device for recording ultrasound scan data in the mouth of the patient is known, which comprises an ultrasound recorder and a support structure. The ultrasound recorder is mounted at rest by the support structure and comprises ultrasound deflection means which are movable. A coupling body is provided, wherein the coupling body is arranged between the ultrasound deflection means and the tooth region or residual tooth region to be scanned when the support structure is placed in the mouth of the patient together with the ultrasound recorder. Moreover, means for applying excitation signals to the ultrasound recorder are provided as well as means for moving the ultrasound deflection means in order thereby to produce an ultrasound wave which passes over at least a part of the tooth region or residual tooth region. EP 2 023 849 furthermore discloses a corresponding method for recording ultrasound scan data in the mouth of a patient by means of the disclosed ultrasound recorder. The method comprises the steps of: placing the support structure for holding the ultrasound recorder into the mouth of the patient, wherein the ultrasound recorder is at rest in relation to the tooth region or residual tooth region while ultrasound scan data is recorded, applying excitation signals to the ultrasound recorder in order to produce an ultrasound wave, moving the ultrasound deflection means in such a way that the ultrasound wave passes over at least one tooth region or residual tooth region, recording waves reflected at the tooth region or residual tooth region by means of the ultrasound recorder or by means of a separate ultrasound receival and providing corresponding reflection signals, making the excitation signals and reflection signals available to an evaluation unit and producing a data record by means of the evaluation unit from the excitation signals and reflection signals with the data record being suitable for transmission to a processing system or an image producing system.

When using such an ultrasound scan device, there is the need to use an intraoral interface structure, which forms the interface between the tooth region or residual tooth region and the respective ultrasound scan device such that the ultrasound scan is obtained with the required high quality. Such an interface structure has to provide a space for coupling medium, which is usually used when performing ultrasound scans.

In the art there are known different systems, which provide a structure for coupling medium. From US 8,231,533 B2 an ultrasound coupling device for transmitting ultrasound waves from ultrasound transducer to a test specimen, especially to a human or animal body, is known, wherein the coupling device has a gel pad for transmitting the ultrasound waves, a support having a transducer side, a body side and an aperture through the transducer side and the body side of the support, the support being provided with an adhesive element on the body side, and the gel pad being received within the aperture such that the gel pad is adapted to contact the human or animal body when the body side of the support contact the human or animal body.

A further device is disclosed in WO2011/163,570 A2. The ultrasound coupling device disclosed therein includes a gel component and a coupling component. The device further comprises a coupling compartment comprising a wall like structure effective for holding the gel component in place, wherein that wall like structure comprises a continuous or substantially continuous side wall, a top surface for interfacing with a subject and a bottom surface and or side surface for interfacing with the ultrasound transducer, and wherein the gel component is contained at least within a portion of the side wall of the wall like structure of the coupling compartment.

From WO 2011/082402 an ultrasound coupling device is known which includes a coupling compartment comprising a chamber having a continuous side wall and an opening on a first end. The continuous side wall is configured to hold a low profile ultrasound transducer within the chamber so that a front ultrasound emitting surface of a low profile ultrasound transducer faces outward toward the chamber opening. From WO2004/062460 a further ultrasound coupling device comprising a pad of solid gel material is known.

An ultrasound coupling device for acoustically coupling an ultrasound transducer with a patient's body is disclosed in US 2008/0033292. The ultrasound coupling device comprises a fluid chamber having an ultrasound transducer interface for placing an ultrasound transducer in contact with water within that fluid chamber. The coupling device also has a patient interface comprising a water permeable membrane. The membrane has an internal membrane surface which is in contact with the water in the water tank and an external membrane surface can be placed in contact with the patient's body. The membrane is specially designed to allow water from inside the water tank to slowly drain or leak to the external membrane surface while such surface is in contact or placed in very close proximity to the patient's skin.

Moreover, a device for providing an interface between medical instrumentation and the patient is disclosed in US 2005/0215901. This interface provides a sterile barrier, acoustic coupler and a thermal insulator between the patient and the medical instrument. In some embodiments, an acoustic coupler interface is used between an ultrasound instrument and the patient.

However, none of the known coupling devices is suitable for being used for scanning a tooth region or residual tooth region in the patient's mouth.

Therefore, an object of the present invention is to provide an intraoral interface structure which provides a coupling between an intraoral scanning device and a tooth region or residual tooth region to be scanned.

This object is solved in the first aspect of the invention by an intraoral interface structure of the aforementioned type, comprising a frame member defining an interior space therein, the frame member being adapted to be placed over the tooth region or residual tooth region, such that the tooth region or residual tooth region is at least partially received in that interior space, wherein the interior space is adapted to be filled with a coupling medium for transmitting ultrasound waves, such that the medium is substantially supported by the frame member, the frame member further comprising an acoustic window and a rim around the acoustic window for forming a receiving portion for a section of an intraoral scanning device, and first and second leg portions extending from the rim for contacting opposing sides of the tooth region or residual tooth region or tissue or bone adjacent to it such that the intraoral scanning device is able to be supported thereat.

The invention is based on the idea, that it is beneficial to provide a coupling medium around the tooth or residual tooth region to be scanned, such that the tooth region or residual tooth region can be scanned substantially from all sides. The scan is carried out such that not only the tooth is scanned but also gingiva and jawbone supporting the respective tooth or residual tooth region. Moreover, also adjacent teeth have to be scanned such that it is possible to provide a high quality restoration based on the scan.

The frame member of the intraoral interface structure according to the present invention has at least two functions. On the one hand, it provides a container, housing or support from the medium, such that the medium is substantially held in place and does not drain away during the scan. The frame provides a support for the medium in this complex and an even scan area such that coupling medium is substantially provided over the whole scanning area. Moreover, the frame provides a support for the respective intraoral scanning device. The rim, which preferably is substantially stiff and rigid, provides a contact area for the intraoral scanning device. The acoustic window is provided such that ultrasound waves from the intraoral scanning device may pass through the acoustic window, through the coupling medium and to the tooth region or residual tooth region to be scanned. The first and second leg portions extend from the rim and are adapted to contact, when received in the mouth of the patient, opposing sides of the tooth region or residual tooth region or tissue or bone adjacent to it such that the intraoral scanning device is able to be supported thereat. A first leg portion e.g. is supported at an outside portion of the tooth region and the second leg is respectively supported at the inside portion of the tooth region. The legs normally contact gingiva tissue and is supported thereat. Thus, the rim and thus the whole frame is carried by the two leg portions extending on opposing sides. In a cross sectional view, the frame may be substantial U-formed.

Additionally, the rim of the frame prevents a tooth contact between the intraoral scanning device and a tooth of the patient, when the patient closes his mouth. Therefore, the intraoral scanning device also is protected by the rim.

In summary, the frame provides a space around the region to be scanned, and the acoustic window which is surrounded by the rim of the frame defines a scan region. When placing in the patient's mouth, the frame is arranged such that the acoustic window is over the tooth region or residual tooth region to be scanned and the frame is filled with coupling medium. Thus, the scan region is properly defined and high quality scans can be obtained.

According to a first preferred embodiment, the leg portions comprise a flexible material for being adjustable to the respective dental anatomy. The respective dental anatomy comprises the anatomy of the tooth region, residual tooth region or a jaw geometry. The first and second leg portions may be bended manually by a dentist, or made out of a flexible, e.g. elastic material, so that a good contact between the respective dental support surface and the neck portions is achieved.

Preferably, the neck portions are biased towards each other. They may be biased towards each other by means of an elastic material the legs are made of or an additional biasing member, as e.g. a clip or a spring. Thus, in the first alternative, the material of the frame is chosen such that the legs are biased and bent towards each other. A dentist has to "open" the leg portions and move them away from each other to place the frame member on the tooth region or residual tooth region. In case a stronger biasing effect is required, also additional elements, as clips, springs or clamps could be used. Such additional elements may be provided within and formed integrally with the frame member.

Moreover, it is preferred that the frame member comprises hand levers extending from the frame member substantially opposing the legs for bending the legs away from each other. Thus, they function substantially as a peg. Having these hand levers, the placement of the frame member for a dentist is greatly simplified. The frame member preferably is closed in the idle state, the legs are contacting each other or at least almost contacting each other. For opening the frame member, the dentist grasps the hand levers and pushes them to open the legs.

According to a further preferred embodiment, the leg portions comprise respective sealing lips at their terminal ends. The terminal ends of the leg portions is the end, opposite the frame member. These portions, the terminal end portions, of the leg portions contact the gingiva or jaw bone of the patient and therefore, it is preferred to provide a sealing lip at this area, for preventing coupling medium draining off the frame member.

In a further preferred embodiment, the frame member comprises at least one adhering element for adhering the frame member to a tooth or residual tooth for fixing the member in the patient's mouth. The adhering element may be formed out of wax, silicone or any biocompatible adhesive. This further leads to a defined position of the frame member and thus to a fixed position for the scanner and in turn to a high quality scan.

Moreover, it is preferred that the frame member has a generally curved shape with a length of covering at least 3 teeth and wherein the acoustic window has a length for covering at least two teeth or residual tooth, and wherein the curvature is generally adapted for matching the curvature of the human jaw. The length is preferably measured along the central axes of curvature in connection to the variation of the dental arch length, width and perimeter as well as the average teeth sizes in the area to be scanned. The length also varies with the ethnic group, gender and age of the patient to be scanned. The term "adapted for matching the curvature of the human jaw" in this instance means, to fit in average adult jaw as it is widely described in the literature and commercially used, as in particular Bonwill WGA. Geometrical and mechanical laws of articulation. Trans Ocont Sot Pa 1885;119:33; Hawley CA. Determination of the normal arch and it application to orthodontia. Dent Cosmos 1905;47:541-52; and Brader AC. Dental arch form related to intraoral forces: PR = C. AM J ORTHOD1972;61:541-61, which are incorporated by reference herein. Preferably the curved shape is formed, according the well-known Bonwill-Hawley form or its derivates, or similar to geometrical shapes such as ellipses, parabolas and catenary curves or mathematic functions such as a cubic spline, conic section or polynomial functions.

In a further development, the frame member comprises a length for enclosing at least three teeth or residual teeth, preferably a quarter of a human set of teeth. In one alternative, the complete lower or upper jaw is enclosed. In such embodiment, the frame is also bent and preferably comprises multiple positions for the respective scanning device, which is preferably marked, to provide defined positions for this scanning device.

In a further preferred embodiment, the frame member comprises at least one internal conduit formed therein, that conduit comprises an inlet opening accessible from outside of the frame member and at least one outlet opening a fluid communication with the interior space, that conduit is usable for filling the interior space with coupling medium while the intraoral interface structure is arranged in a patients mouth. The inlet opening preferably is provided with a connector such that a supply of coupling medium may be attached to the frame member. Preferably a plurality of outlet openings is provided. This allows that a dentist may fill the coupling medium through the conduit, which has one inlet in multiple outlets, such that the coupling medium is evenly filled into the interior space defined by the frame member. Preferably, the outlet openings are in the area of the terminal ends of the respective leg portions. Thus, the interior space is filled from the jaw bone into the direction of the top of the tooth, such into the direction of the acoustic window and a dentist can easily recognize, if air bubbles are present in the coupling medium.

Preferably, the conduit branches within the frame member and terminates at a plurality of spaced apart outlet openings. Preferably, the outlets are arranged in the area of interdental spaces and the coupling medium is first guided to the interdental spaces to push air away.

According to further preferred embodiment, the intraoral interfaces structure comprises a contact indicator for indicating a correct engagement between the receiving portion and the section of the intraoral scanning device. Such a contact indicator in one embodiment is adapted to provide a signal when in contact with a corresponding contact area of a head portion of an intraoral scanning device.

In a further embodiment, the contact indicator may incorporate an electromagnetic contact, an inductive element, an optical element or a mechanical element, such as a pin being received in a corresponding recess or an acoustic element, which emits a click sound or the like, when engaged.

In a further preferred embodiment, the intraoral interface structure comprises a heating device for heating a coupling medium within the interior space. Preferably the coupling medium has the same temperature as the surrounding tissue of the patient's mouth, such that impedance matching and transmission of ultrasound waves is improved. In the state of the art, normally a dentist waits a couple of minutes, such that the medium heats up itself, however it is not a desirable position for a patient to await a couple of minutes with such an intraoral interface structure in his mouth. Therefore, the heating device is provided, to heat up the medium accordingly. Moreover, with such a heating device, the medium can be more accurately heated up. The heating device may comprise a ring shaped heating element, running around the frame member. Moreover, the heating device may comprise heating wires integrally attached to the leg portions.

In a further preferred embodiment, the intraoral interfaces structure comprises a sensor arrangement comprising at least one centre device attached to set frame member for measuring at least one feature of that frame member and or a coupling medium within the interior space.

In a first further development of this aspect, the sensor arrangement comprises a temperature sensor for measuring a temperature of a coupling medium within the interior space. This embodiment is particularly preferred in combination with a heating device. The heating device may heat up the coupling medium and the temperature sensor of the sensor arrangement measures the temperature of the coupling medium. Thus, the temperature of the coupling medium can be controlled to be in a respective temperature range and when this temperature is reached, the heating device can be switched off.

In a further preferred embodiment, the sensor arrangement comprises a force sensor measuring a force acting on the frame member. In particular, such a force is a force due to a biting action of the patient. The patient shall fix the intraoral scanning device to the intraoral interface structure during a scanning operation by biting on the device. This force preferably is measured and the force sensor is connected with a computing device and this force is output. When the force exceeds a predetermined pressure, preferably a warning signal is emitted. This helps to gain high quality scans, since the frame member will still be in position and not displaced by the biting action.

In a further preferred embodiment, the sensor arrangement comprises an accelerometer for determining movements of the frame member. Thus, it can be determined whether the patient moves or not and therefore it can be determined whether the placement of the frame member is still accurate.

In a further preferred embodiment, the intraoral interface structure comprises a holding arm, fixedly attached to the frame member, the holding arm being adapted to be attached to an external support. E.g. the holding arm may be adapted to be attached to a dentist chair. The holding arm holds the frame member in a predetermined relation to e.g. the dentist chair or any other external support, such as a roller support, and the patient moves his head towards the intraoral interface structure. Thus, the relation of the frame member and the scanning device is always known, however the patient may move. In such embodiment, accelerometers are not necessary.

According to a further aspect of the invention, the problem named in the introductory portion is solved by a system for scanning a patient's mouth, comprising: an intraoral scanning device for recording ultrasound scan data in the mouth of the patient, the device comprising: a housing having a proximal portion and a distal portion, the distal portion being adapted for placement within the patient's mouth, a high frequency sonic head comprising at least one transducer for scanning a covered or non-covered tooth region or residual tooth region and mounted within the distal portion of the housing, and means for applying excitation signals to the sonic head in order thereby to produce an ultrasonic wave which passes over at least part of the tooth region or residual tooth region such that the sonic head receives ultrasound waves reflected by the tooth region or residual tooth region to be scanned, wherein the sonic head is movable at least in a first and a second degree of freedom, and further comprising a drive assembly, arranged within the proximal portion of the housing for driving the sonic head in the first and the second degree of freedom such that the sonic head is movable in a plane for applying the ultrasonic wave to a predetermined region of the tooth region or residual tooth region; and the system further comprising an intraoral interface structure according to any of the above described preferred embodiments of an intraoral interface structure according to the first aspect of the invention.

It shall be understood that the intraoral interface structure according to the first aspect of the invention and the system according to the second aspect of the invention comprise similar and identical preferred embodiments which are in particular described in the dependent claims. Therefore, reference is made to the above description as to the preferred features and their effect.

The intraoral interface structure preferably is arranged at the distal portion of the housing and in contact therewith, such that the ultrasonic wave which is emitted by the at least one transducer passes through the acoustic window of the intraoral interface structure and to the tooth region to be scanned.

The intraoral scanning device is formed as a handheld device. The housing comprises a distal portion which is to be placed inside the patient's mouth when a recording process is carried out. Preferably the distal portion of the housing is formed such that the patient may bite on it for fixing the intraoral scanning device during data acquisition. The high frequency sonic head is mounted within the distal portion of the housing and, thus, is adjacent to the tooth region or residual tooth region to be scanned during the recording process. This sonic head is moved by the drive assembly in the first and second degree of freedom such that the sonic head is moved in a plane over the tooth region or residual tooth region to be scanned. Therefore, it is possible to use a single transducer even though also an array could be used within the scope of the invention. The benefit of an array, in general, is that it is not necessary to move it in a plane, because it consists of a plurality of transducers. On the other hand, a high-frequency single transducer has the benefit that it has a high focus in depth and, thus, can record ultrasound scan data with high accuracy. Additionally, single transducers are more cost effective than transducer arrays. The transducer is preferably adapted to transmit ultrasonic waves with a frequency in the range of 20 to 100 MHz, preferably 40 to 100 MHz, more preferably 40 to 60 MHz.

While the distal portion and the transducer are adapted for placement within the patient's mouth, the proximal portion of the housing preferably comprises a handle so that an operator can hold and place the housing to position the sonic head for a recording operation. Preferably the patient fixes the distal portion by biting. Alternatively, an additional fixing apparatus such as a support arm or the like is provided. The tooth region or residual tooth region to be scanned includes all dental hard and soft tissue structures, such as teeth, bone, gingiva, nerves and the like. The expression "residual tooth" refers to a part of a tooth which e.g. has already been prepared for a dental reconstruction. In the following, when reference is made to a "tooth" or a "residual tooth", it shall be understood that both is meant, even though only one term is used. When reference is made to a "covered tooth region" or "covered residual tooth region", this means that the tooth region or residual tooth region is covered with soft tissue, e.g., gingiva, saliva, blood, or sulcular fluids. The term "uncovered tooth region" refers to the tooth or bone itself.

Preferably, the intraoral scanning device comprises or is connected to a processing unit for processing the recorded ultrasound scan data for enhancing their depth of focus and/or for reconstructing a 3D model. The processing unit may be provided within the housing, preferably, in the distal portion. In a preferred embodiment, the processing unit is provided external to the housing and connected to the housing, in particular, to the sonic head, via a cable or wireless. In a preferred solution the processing unit is provided as a separate unit on a movable support device, such as a roller carriage. The processing unit is preferably arranged as close as possible to the sonic head, such that damping and noising of the recorded signal is reduced by a short wiring between the sonic head and the processing unit. In one embodiment, the processing unit is provided in a separate housing which might be provided at a head rest of a dental chair when the intraoral scanning device is used. Alternatively, the patient may hold this controller housing in his hands during the scanning process.

According to a third aspect of the invention, the problem stated in the introductory portion is further solved by a method for scanning a tooth region or residual tooth region comprising the steps: placing an intraoral interface structure according to any of the above described preferred embodiments of an intraoral interface structure according to the first aspect of the invention, in a patient's mouth such that a tooth region or residual tooth region to be scanned is at least partially received in said interior space; filling the interior space with a coupling medium for impedance matching; engaging the receiving portion with a section of an intraoral scanning device; and scanning the tooth region or residual tooth region by emitting ultrasound waves to the tooth region or residual tooth region.

Again, it shall be understood that the method according to the third aspect of the invention and the intraoral interface structure of the first aspect of the invention comprise similar and identical aspects which are particularly described in the dependent claims. In so far, reference is made to the above description of the intraoral interface structure of the first aspect of the invention as well as their features and the respective technical effects.

In the following, the invention is described in more detail with reference to the accompanying drawings, in which:
- Fig. 1: shows a schematic perspective view of a lower jaw bone with an intraoral interface structure according to a first embodiment;
- Fig. 2: shows a lower jaw bone with an intraoral interface structure according to a second embodiment;
- Fig. 3: shows a schematic perspective view of a lower jaw bone with an intraoral interface structure according to a third embodiment;
- Fig. 4: shows a lower jaw bone with an intraoral interface structure according to a fourth embodiment;
- Fig. 5: shows the embodiment of Fig. 2 in a further perspective view;
- Fig. 6: shows a detail of Fig. 5;
- Fig. 7: shows the view of Fig. 5 with a further embodiment of the invention;
- Fig. 8: shows a detail of Fig. 7;
- Fig. 9: shows a perspective view of a further embodiment of the intraoral interface structure;
- Fig. 10: shows a different view of the embodiment of Fig. 9;
- Fig. 11: shows a further embodiment of intraoral interface structure;
- Fig. 12: shows the embodiment of Fig. 11 in a further state;
- Fig. 13: shows a schematic perspective view of a lower jaw bone with an intraoral interface structure according to a further embodiment;
- Fig. 14: shows a further perspective view of an intraoral interface structure according to a further embodiment;
- Fig. 15: shows the embodiment of Fig. 14 with additionally a coupling medium supply;
- Fig. 16: shows a further embodiment of the intraoral interface structure for engagement with an intraoral scanning device;
- Fig. 17: shows a further view of the embodiment of Fig. 16;
- Fig. 18: shows a further embodiment of the intraoral interface structure for engagement with an intraoral scanning device;
- Fig. 19: shows a further view of the embodiment of Fig. 18;
- Fig. 20: shows the intraoral interface structure arranged in the patient's mouth and in conjunction with the intraoral scanning device;
- Fig. 21: shows a further embodiment of the intraoral interface structure;
- Fig. 22: shows a perspective partially cut-out view of an intraoral scanning device; and
- Fig. 23: shows the intraoral scanning device of fig. 22 without housing and only base plate;

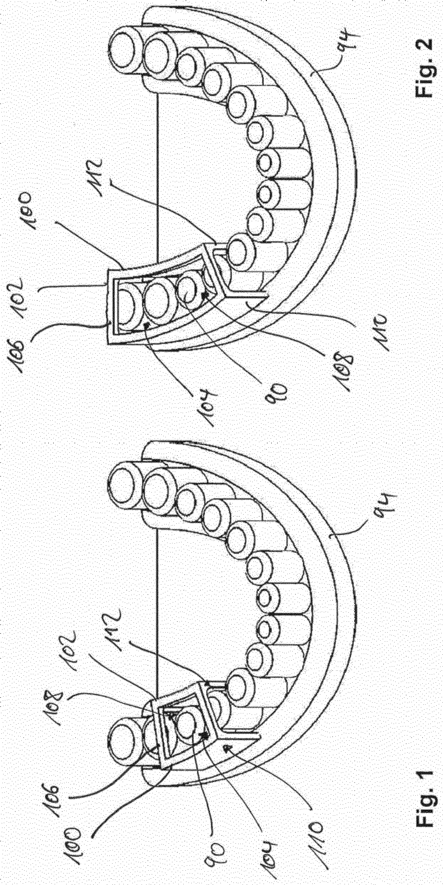

According to Figs. 1 to 4, four different embodiments of an intraoral interface structure 100 is shown. The intraoral interface structure 100 is placed on a lower jaw 94 of a patient to scan a tooth region 90. A residual tooth region in this embodiment is not shown, however could be a prepared tooth, which has been prepared for receiving a crown afterwards. The tooth region, including the neighboring teeth, which have to be scanned, so that a dental restauration can be prepared.

The intraoral interface structure 100 comprises a frame member 102 defining an interior space 104 therein. The interior space 104 at least partially receives the tooth region 90 to be scanned.

The frame member 102 moreover comprises a rim 106 surrounding an acoustic window 108 which in these embodiments (Figs. 1 - 4) is formed as a through-hole opening.

The frame member moreover comprises first and second leg portions 110, 112, wherein the first leg portion 110 is provided for the outside of the jaw 94 and the second leg portion 112 for the inner portion of the jaw 94.

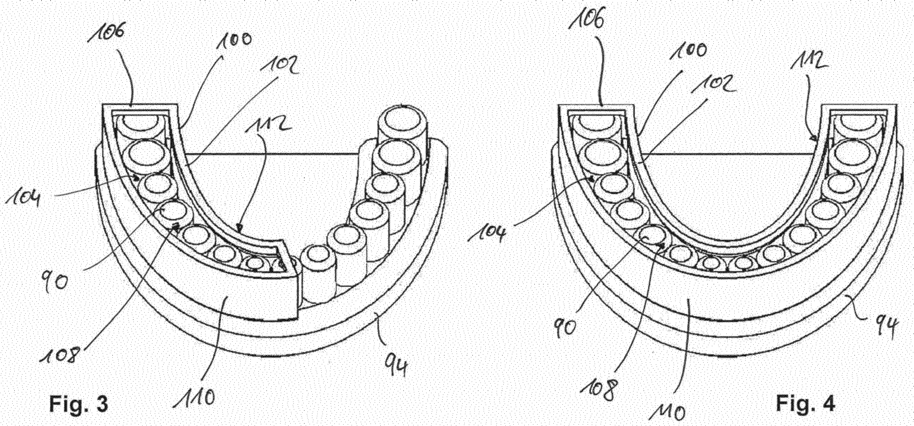

The described four embodiments of Figs. 1 to 4 differ in the length of the respective frame member 102. While the frame member 102 according to Fig. 1 is provided for only one or two teeth, the frame member 102 according to Fig. 2 is provided for a plurality of teeth, in this particular embodiment three teeth. Fig. 3 shows an embodiment in which the frame member 102 is provided for a quarter of the set of teeth of the indentation that is half of a lower jaw 94. Fig. 4 shows an embodiment in which the frame member 102 encloses all teeth of the lower jaw 94. The respective length of the frame member 102 is measured along the row of teeth.

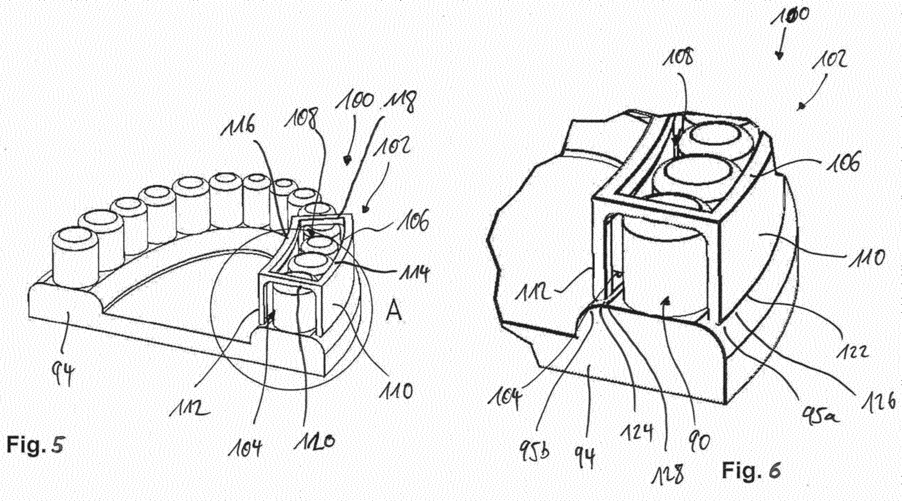

Fig. 5 shows a perspective view of the embodiment of Fig. 2, from the back side of the jaw 94 to more clearly explain the structure of frame member 102. With respect to Figs. 5 and 6, the frame member 102 comprises a rim 106, which has a substantially rectangular form having first and second side sections 114, 116 and first and second forward and backward sections 118, 120. The first and second leg portions 110, 112 extend from the side portions 114, 116 of the rim 106. The forward and backward sections 118, 120 of the rim 106 are free, such that the frame member 102 can be placed over the tooth region or row of teeth.

Each of the first and second leg portions 110, 112 comprise a terminal end 122, 124, seating on a respective section 95a, 95b of the gingiva of the jaw 94. Thus, the frame member 102 and also the rim 106 and therefore the acoustic window 108 are fixedly supported over the tooth region 90. The terminal ends 122, 124 are preferably provided with a sealing lip 126, 128 for a good sealing contact between the leg portions 110, 112 and the respective gingiva surface portions 95a, 95b.

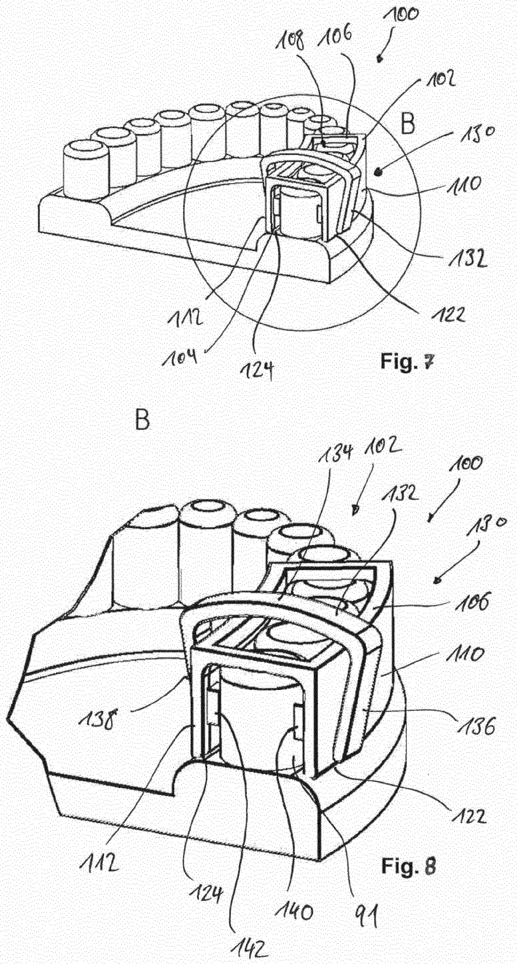

For an even better sealing and seating of the frame member 102, the intraoral interface structure may comprise biasing means 130 (see Figs. 7 - 12).

According to the embodiment shown in Figs. 7 and 8 the biasing means 130 comprise a clip 132, engaging both leg portions 110, 112 so as to push the terminal ends 122, 124 together, so that a sealing effect is increased. The clip 132 comprises a slightly rounded back 134 and two opposing legs 136, 138 extending substantially rectangular from the back 134. The legs 136, 138 have a length, such that their tips may contact the terminal ends 122, 124 and the back 134 is still above and not contacting the rim 106.

In the embodiment shown in Figs. 7 and 8, the intraoral interface structure 100 additionally comprises two adhering elements 140, 142, which in this embodiment are formed as wax elements. The adhering elements 140, 142 are attached to inner portions of the leg portions 110, 112 and in contact with a tooth 91, so that the frame member 102 is fixed by an adhesive force to the tooth 91. This further increases positioning of the intraoral interface structure 100.

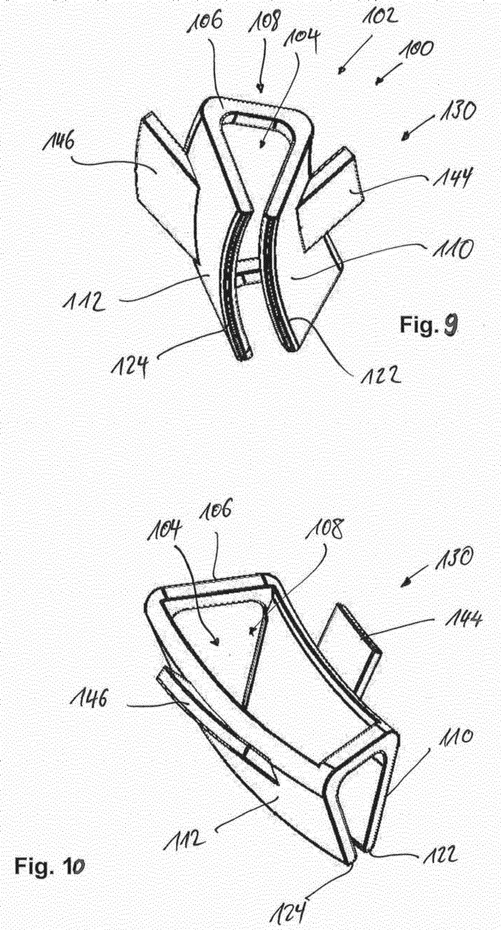

The embodiment shown in Figs. 9 and 10 shows a frame member 102 having biasing means 130. The biasing means 130 according to this embodiment (Figs. 9 and 10) comprise that the material, the frame member 102 is made of, is elastic, and the leg portions 110, 112 are biased towards each other, such that the terminal ends 122, 124 are in close proximately to each other. Thus, in cross-section, the frame member 102 according to this embodiment is substantially V-shaped or triangle-shaped. For placing the frame member 102 on a tooth region 90, it is necessary to move away the terminal end 122, 124 from each other such that the interior space 104 is accessible from the lower side of the frame member 102. To simplify the opening of the frame member 102, according to this embodiment, two hand levers 144, 146 are attached to the first and second leg portions 110, 112 close to the rim 106 and extending, as seen from the terminal ends 122, 124, above the rim 106. Moreover, they extend in an acute angle to the sides. For opening the frame member 102, the hand levers 144, 146 are pushed together by a dentist, such that the leg portions 110, 112 pivot, to move the terminal ends 122, 124 away from each other.

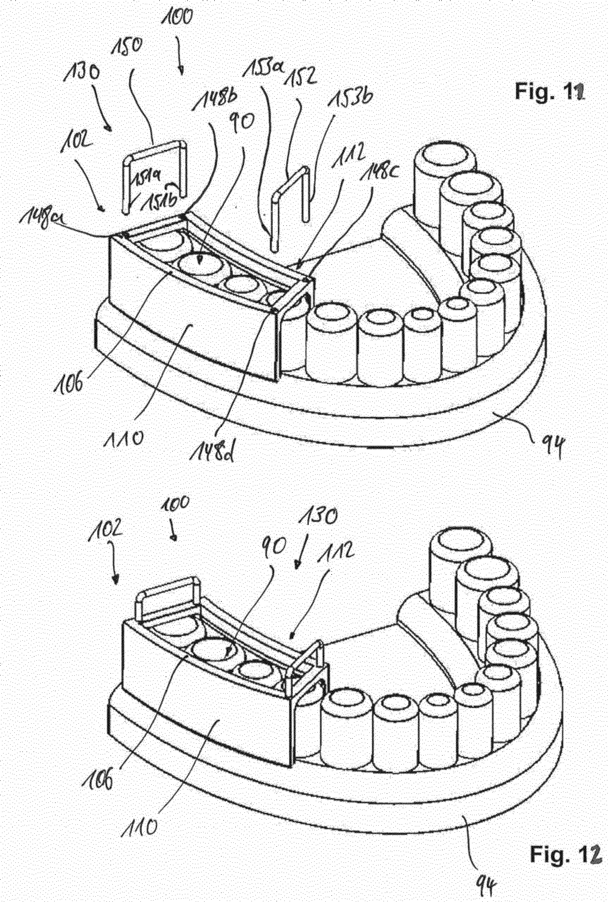

Figs. 11 and 12 illustrate a further example of biasing means 130. In this example, the frame member 102 comprises four recesses 148a, 148b, 148c, 148d, provided at edges of the substantially rectangular frame 106. The recesses 148a-148d are formed as bores and extend into a direction of the plane of the respective leg portions 110, 112. The recesses 148a-148d are adapted to receive clamps 150, 152, which comprise longitudinal extending legs 151a, 151b, 153a, 153b. The clamps 150, 152 can be seated within the recesses 148a-148d (see Fig. 12) to provide additional stability to the frame member 102. The frame member 102 is made of a flexible plastic material and the clamps 150, 152 can be seated within the recesses 148a-148d to provide stability to the leg portions 110, 112, such that they do not deflect outwardly.

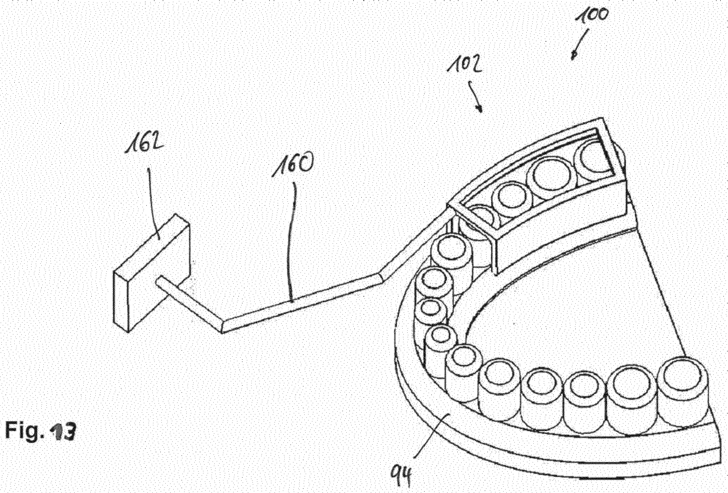

A further possibility for positioning the frame member 102 correctly in the patient's mouth is illustrated in Fig. 13. The frame member 102 is coupled and attached to a holder arm 160, which in turn is fixed to a base 162. The base 162 might be a support, as a roller support, or a dentist's chair. The frame member 102 in this embodiment is fixed in its spatial position and the patient has to bring its jaw 94 into contact with the respective frame member 102.

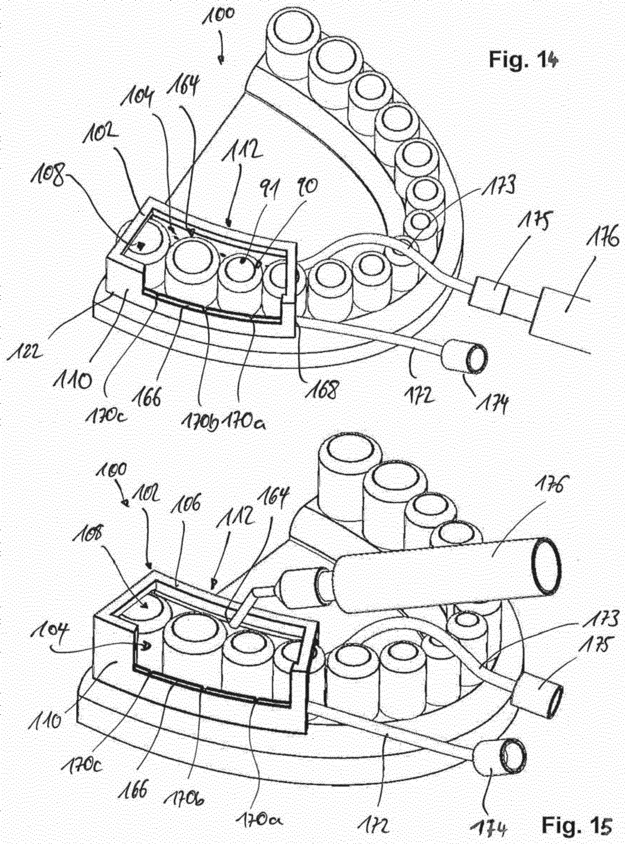

The frame member 102 is adapted to receive a coupling medium 164 within the interior space 104. The coupling medium 164 is necessary to provide a good transmission of ultrasound waves from an intraoral scanning device comprising an ultrasound transducer. The way to fill the interior space 104 is illustrated in Figs. 14 and 15. In Figs. 14 or 15, the interior space 104 is at least partially filled with a coupling medium 164. The frame member 102 comprises first and second conduits 166 (only one can be seen in Figs. 14 and 15, which run within the leg portions 110, 112). In Figs. 14 and 15, the leg portion 110 is partially cut away to show the conduit 166. It shall be understood that also the leg portion 112 comprises an identical formed conduit. The conduit 166 comprises an inlet 168 and a plurality of outlets 170a, 170b, 170c, which are close to the terminal end 122, such that they are close to the roots of the respective teeth 91 of the tooth region 90.

The inlet 168 is coupled with a hose 172 having a connector 174. The connector 174 is attachable to a cartridge for coupling medium 164. In Fig. 14, a second conduit 173, which is coupled with the conduit of the leg portion 112 is attached via its connector 175 to a cartridge 176. In operation, the cartridge 176 may be coupled sequentially to connectors 174, 175, such that the interior space 104 is completely filled. Coupling medium 164 will flow out of the outlets 170a, 170b, 170c, and the interior space 104 is filled from the lower portion to the upper end until coupling medium 164 drains off the acoustic window 108.

For additionally filling the upper space, it might also be necessary to use the cartridge 176 to directly fill coupling medium 164 from above through the acoustic window 108, as it is illustrated in Fig. 15.

The conduit 166 may also be used to draw medium out of the interior space 104, e.g. after completing a scanning operation or in case excess coupling medium has been filled into the interior space.

The coupling medium may comprise or consist of an impedance matching medium having low damping characteristics. The intraoral interface structure is used to keep the coupling medium, which typically is liquid having, however, a relatively high viscosity in place. The coupling medium should be provided around the tooth region or residual tooth region to be scanned during a scan process. The coupling medium is used for impedance matching such that an energy-loss in the acoustic wave transmission is minimized. For recording high quality and high accuracy ultrasound scan data, it is important that the ultrasonic wave transmitted from the transducer is irritated as less as possible by any medium between the transducer and the tooth region or residual tooth region to be scanned. This increases the accuracy of the recorded ultrasound scan data.

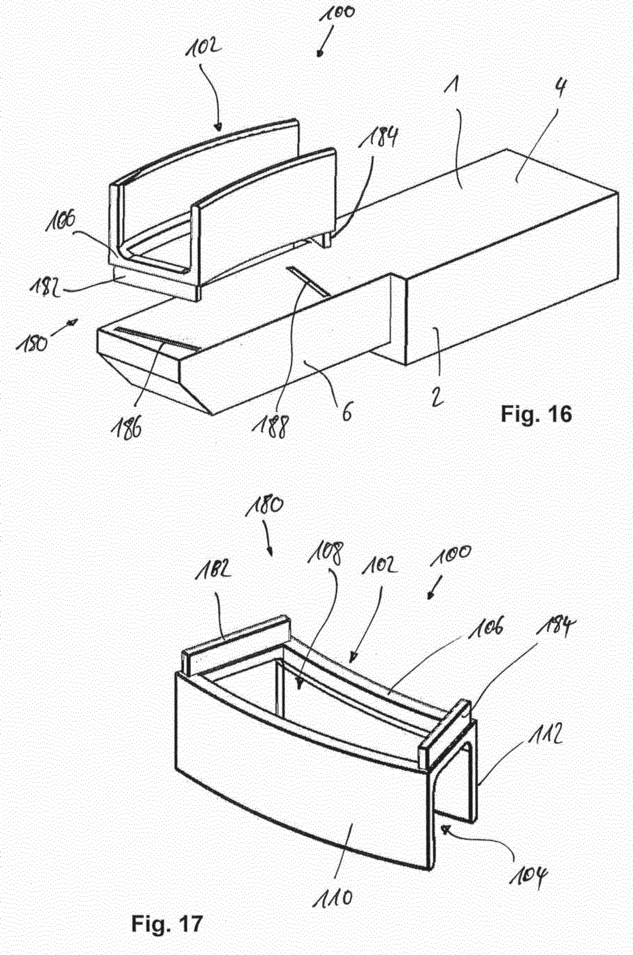

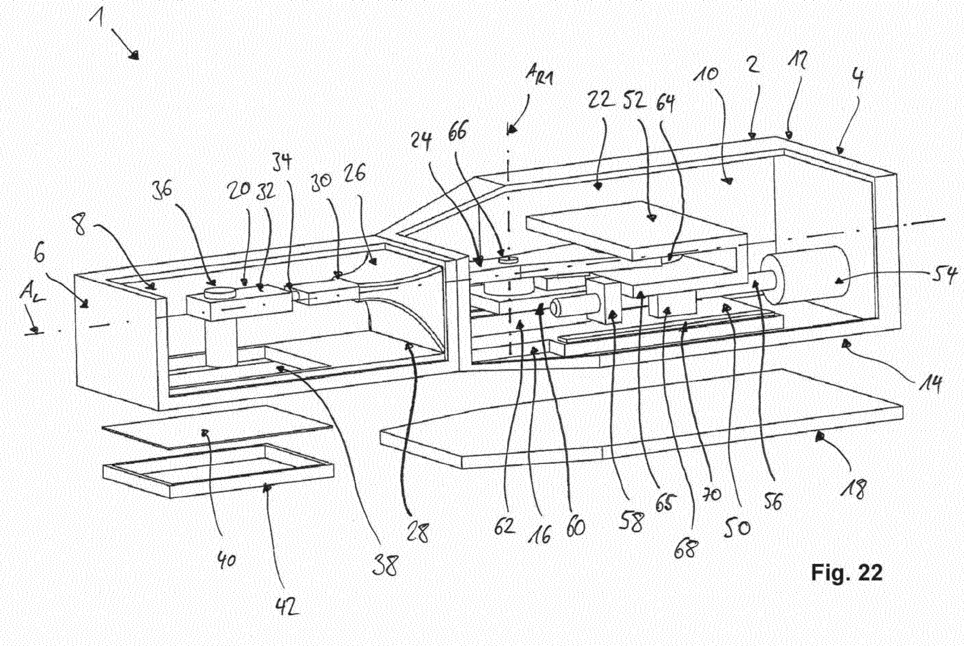

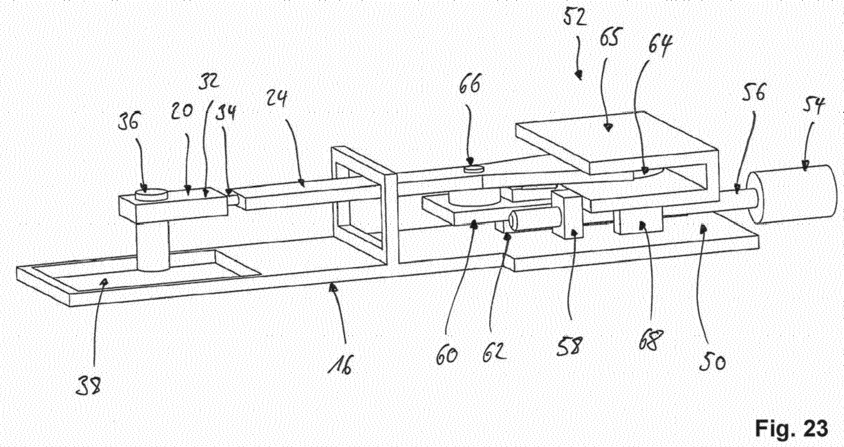

Figs. 16 to 19 disclose further embodiments, in which additional engagement means 180 are provided for the intraoral interface structure for a proper engagement with the respective section of an intraoral scanning device 1. The intraoral scanning device 1 comprises a proximal portion 4 and a distal portion 6, wherein the distal portion 6 comprises the ultrasound transducer 36 (see Figs. 22, 23). Therefore, the distal portion 6 of the housing 2 of the intraoral scanning device 1 has to be connected with the rim 106 of the intraoral interface structure 100. The rim 106, according to this embodiment (Figs. 16 and 17), is provided with two protruding lips 182, 184, extending in the opposite direction of the leg portions 110, 112 from the rim 106. The lips 182, 184 are substantially rigid and adapted to be received in respective recesses 186, 188 at the distal portion 6. Therefore, a form-fitting connection between the intraoral interface structure 100 and the intraoral scanning device 1 is provided and a defined relationship between both elements is achieved.

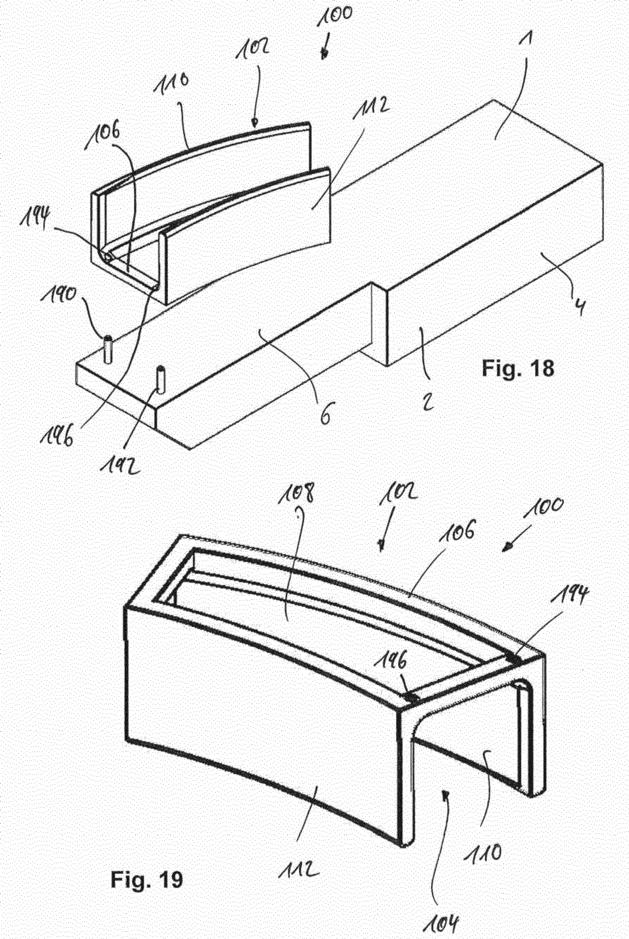

An alternative embodiment hereto is shown in Figs. 18 and 19. In this embodiment (Figs. 18 and 19) the distal portion 6 of the housing 2 of the intraoral scanning device 1 is provided with two pins 190, 192, which are adapted to engage respective recesses 194, 196 formed at the rim 106 of the frame member 102. The recesses 194, 196 are formed as through-holes so that the pins 190, 192 may extend to the inner space 104. Alternatively holes 148a, 148b, 148c, 148d can be used.

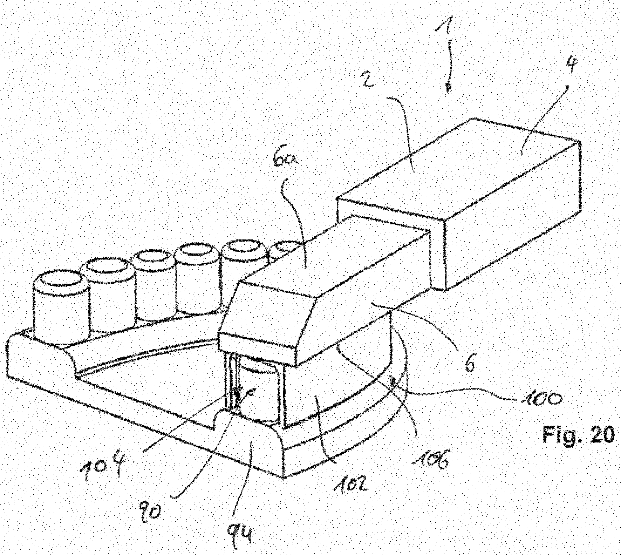

In operation, the distal portion 6 of the housing 2 of the intraoral scanning device 1 is seated on the rim 106 of the intraoral interface structure 100, which in turn is placed over the tooth region 90 to be scanned of a lower jaw 94 such that the tooth region 90 extends into the inner space of the frame member 102. This arrangement is shown in Fig. 20. The patient may bite on the upper surface 6a of the distal portion 6 to fix the arrangement and to provide a stable position of the intraoral scanning device 1.

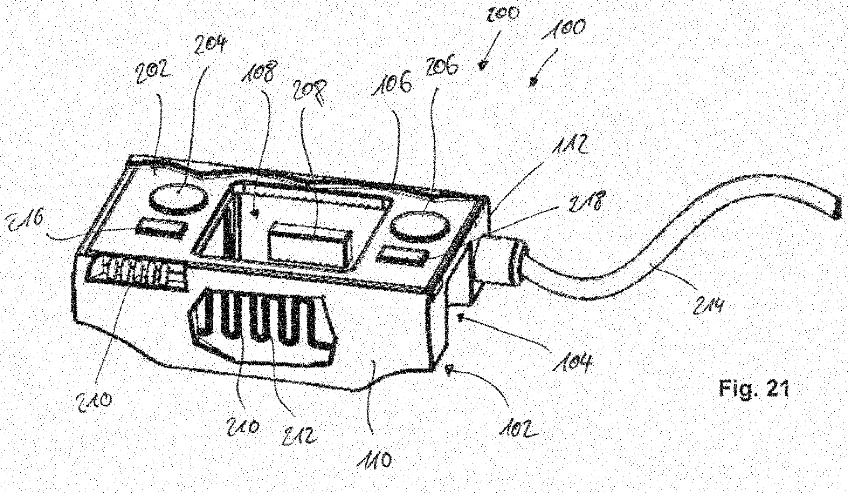

Fig. 21 illustrates a further embodiment of an intraoral interface structure 100. The intraoral interface structure 100, according to this embodiment, comprises a sensor arrangement 200, which will now be described. The sensor arrangement 200 comprises a circuit board 202, which is integrally formed with the frame member 102. The frame member 102, as already described with respect to the previous embodiments, comprises a rim 106 and two opposing leg portions 110, 112. In this embodiment (Fig. 21), the thickness of the single elements is bigger to accommodate the elements of the sensor arrangement 200.

The circuit board is provided with an accelerometer 204 and a force sensor 206. The accelerometer 204 is adapted to measure movement of the intraoral interface structure 100 and the force sensor 206 is adapted to measure force acting on the frame 206, e.g. when the patient bites on surface 6a (see Fig. 20).

Moreover, the sensor arrangement comprises a temperature sensor 208 arranged at an inside portion of the leg portion 212 to measure temperature of a coupling medium within the interior space 104. For heating the coupling medium within the interior space, the intraoral interface structure 100, according to this embodiment (Fig. 21), comprises a heating device 210, comprising heating wires 212, which run within the material of the leg portion 110.

For transmitting electrical energy and data, the circuit board 202 is provided with an electrical connector 214, which in turn may be connected to a computer, or an intraoral scanning device 1.

Moreover, according to this embodiment (Fig. 21), the circuit board 202 is provided with two contacts 216, 218. These contacts 216, 218 act together with corresponding contacts at the distal portion 6 of the intraoral scanning device 1. When the contact is closed, it is determined that the placement of the intraoral scanning device 1 and the intraoral interface structure 100 is correct. If the contact is interrupted, it is an indicator that the intraoral scanning device 1 and the intraoral interface structure 100 do not match and that a replacement is necessary.

An intraoral scanning device 1 which may be used in conjunction with the intraoral interface structure 100 as described above, comprises a housing 2 with a proximal portion 4 and a distal portion 6, wherein the distal portion 6 is adapted to be placed within the patient's mouth (see also Fig. 21). The housing 2 comprises a wet area 8 and a dry area 10, wherein the wet area 8 is provided in the distal portion 6 of the housing 2 and the dry area 10 in the proximal portion 4 of the housing 2. The proximal portion 4 is suited to be gripped by an operator and may comprise an additional handle or the like. With respect to Fig. 22, the housing 2 comprises an upper portion 12 and a lower portion 14, wherein the lower portion 14 is formed by a base plate 16 (see Fig. 23). The base plate 16 runs from the distal portion 6 to the proximal portion 4 of the housing 2 and provides structural support. As shown in Fig. 22, the base plate 16 within the proximal portion 4 is provided with a shielding plate 18 which might be - as well as all housing parts - formed out of a grippable rubber material, so that the operator does not have to touch a slippery metallic surface provided by the base plate, but can easily grip and handle the intraoral scanning device 1.

The intraoral scanning device 1 moreover comprises a high frequency sonic head 20 and a drive assembly 22 for driving the sonic head 20 in a first and second degree of freedom (see, in particular, Fig. 23). The drive assembly 22 comprises a rotor bar 24 which is rotatable about a first rotational axis A_{R1}. The rotor bar 24 extends from the proximal portion 4 to the distal portion 6 and from the dry area 10 to the wet area 8. Moreover, the rotor bar 24 extends through a sealing membrane 26 which is provided between the distal portion 6 and the proximal portion 4 and seals the wet area 8 from the dry area 10. Membrane 26 is shown to be funnel-shaped. This is not necessary but membrane 26 could also be formed having a bellow or bag which allows lateral movement of the rotor bar 24 with respect to the housing 2 in the portion where the membrane 26 is provided, and also allows movement perpendicular to the rotational axis A_{R1} along the longitudinal axis A_{L} of the scanning device. Membrane 26 is fixedly and sealingly connected with its outer rim 28 to the housing 2 and with its inner rim 30 to the rotor bar 24.

The sonic head 20 comprises a head body 32 which is connected via a pivotable connection 34 to the rotor bar 24. The pivotable connection 34 allows swiveling the sonic head 20 about axis A_{R2} which in Fig. 22 is identical with axis A_{L}. Axis A_{R2} is the longitudinal axis of the rotor bar 24. The head body 32 according to the embodiment shown in Fig. 22 carries a single transducer 36 which projects downwardly in Fig. 22. The single transducer 36 is formed as a high frequency transducer which is able to excite ultrasonic waves with a frequency of 50 MHz and is also able to receive the reflected waves.

To allow pass-through of the ultrasonic waves, housing 2 comprises an opening 38 which is formed in the distal portion 6 of the housing 2. The opening 38 is closed with an acoustic window 40 which is held in place and is biased by means of a frame 42. In Fig. 22, the acoustic window 24 and the frame 42 are shown in an exploded view but are mounted within the opening 38. The frame protects the acoustic window 40 and also biases the acoustic window 40 which is formed by a foil element having a low impedance for ultrasonic waves and matches the impedance of the coupling medium. This results in a low reflexion factor and no impedanz difference.

The drive assembly 22 is formed as a serial kinematic and comprises a linear actuator 50 for driving the sonic head 20 in a first degree of freedom and a rotational actuator 52 for driving the sonic head 20 in a second degree of freedom. The first degree of freedom is a linear degree of freedom along the axis A_{L} and the second degree of freedom is a rotational degree of freedom about the rotational axis A_{R1}. The linear actuator 50 comprises a linear motor 54 driving a spindle 56. The linear motor 54 is mounted at the base plate 16. The spindle 56 acts together with a nut 58 which is attached to a body 60 of the rotational actuator 52. The body 60 of the rotational actuator 52 is guided in a linear bearing 62 which allows linear movement of the body 60 along the linear bearing 62 defining a linear axis by means of actuating the linear drive 54. When the linear drive 54 is actuated, the spindle 56 is rotated and the nut 58 is moved back and forth along the spindle 56 and, thus, the body 60 is moved back and forth accordingly.

The rotational actuator 52 is formed as a voice coil drive. It comprises the body 60 which is substantially U-shaped in cross section and supported in the linear bearing 62 and connected to the nut 58. The rotational actuator 52 comprises a coil 64 which is supplied with current for inducing an electromagnetic field and a corresponding magnetic field generated by a ferromagnetic circuit 65 having neodymium magnet, provided within the body 60. The coil 64 is fixed to the rotor bar 24 which is supported on a rotational bearing 66 rotatable about axis A_{R1}. The bearing 66 is carried by the body 60. The body 60 thus carries the bearing 66 and the ferromagnetic circuit 65.

The rotational actuator 52, in particular the ferromagnetic circuit65 is provided with a sensor 68 which is movable together with the rotational actuator relative to the base plate 16. The base plate 16 is provided with a scale 70 which is read by the sensor 68.

A second sensor 72 is provided on the body 60 of the rotational actuator 52 and reads a scale 74 which is attached to the rotor bar 24. By means of the two sensors 68, 72, the position of the sonic head 20 can be measured.

As can be inferred from the figures, the section of the rotor bar 24 from the sonic head 20 to the bearing 66 is longer than the section from the bearing to the coil, preferably by 10% to 50%. This arrangement helps to reduce the velocity of the active in respect to the passive element of the rotational encoder 68, 70 leading to a robust position measurement. Repeatability in view of positioning the sonic head 20 is important. Alternatively, when the rearward section of the arm 24 is longer, or the encoder 68, 70 is placed in the distal portion of the housing 2, the arm 24 provides a leverage effect and thus acts as a measurement amplifier.

## Claims

1. An intraoral interface structure (100) for arrangement between an ultrasound transducer (36) of an intraoral scanning device (1) and a tooth region (90) or residual tooth region to be scanned, and for transmitting ultrasound waves from the transducer (36) to the tooth region (90) or residual tooth region, the intraoral interface structure (100) comprising:
a frame member (102) defining an interior space (104) therein,
the frame member (102) being adapted to be placed over the tooth region (90) or residual tooth region, such that the tooth region (90) or residual tooth region is at least partially received in said interior space (104), wherein the interior space (104) is adapted to be filled with a coupling medium (164) for impedance matching,
the frame member (102) comprising an acoustic window (108) and a rim (106) around the acoustic window (108) for forming a receiving portion for a section of an intraoral scanning device (1), and first and second leg portions (110, 112) extending from said rim (106) for contacting opposing sides of the tooth region (90) or residual tooth region or tissue or bone adjacent to it such that the intraoral scanning device (1) is able to be supported thereat.

2. The intraoral interface structure according to claim 1, wherein the leg portions (110, 112) comprise a flexible material for being adjustable to the respective tooth region (90) or residual tooth region.

3. The intraoral interface structure according to claim 2, wherein the leg portions (110, 112) are biased towards each other.

4. The intraoral interface structure according to claim 3, wherein the frame member (102) comprises hand levers (144, 146) extending from the frame member (106) substantially opposing said leg portions (110, 112) for bending the leg portions (11, 112) away from each other.

5. The intraoral interface structure according any of the preceding claims, wherein the leg portions (110, 112) comprise respective sealing lips (122, 124) at their terminal ends.

6. The intraoral interface structure according any of the preceding claims, wherein the frame member (102) comprises at least one adhering element (140, 142) for adhering the frame member (102) to a tooth (91) or residual tooth for fixing the frame member (102) in a patient's mouth.

7. The intraoral interface structure according any of the preceding claims, wherein the frame member (102) has a generally curved shape with a length of covering at least three teeth and wherein the acoustic window (108) has a length for covering at least two teeth (91) or residual teeth, and wherein the curvature is generally adapted for matching the curvature of a human jaw (94).

8. The intraoral interface structure according to claim 7, wherein the frame member (102) comprises a length for enclosing at least three teeth or residual teeth, preferably a quarter of a human set of teeth.

9. The intraoral interface structure according any of the preceding claims, wherein the frame member (102) comprises at least one internal conduit (166) formed therein, said conduit (166) comprises an inlet opening (168) accessible from outside of the frame member (102) and at least one outlet opening (170a, 170b, 170c) in fluid communication with the interior space (104), said conduit (166) is usable for filling the interior space (104) with coupling medium (164) while the intraoral interface structure (100) is arranged in a patient's mouth.

10. The intraoral interface structure according claim 9, wherein said conduit (166) branches within said frame member (102) and terminates at a plurality of spaced apart outlet openings (170a, 170b, 170c).

11. The intraoral interface structure according to claim 9 or 10, wherein said outlet openings (170a, 170b, 170c) are arranged at said leg portions (110, 112), in particular adjacent to terminal ends of the leg portions (110, 112).

12. The intraoral interface structure according any of the preceding claims, comprising a contact indicator for indication a correct engagement between said receiving portion and the section of an intraoral scanning device (1).

13. The intraoral interface structure according any of the preceding claims, comprising a heating device (210) for heating a coupling medium (164) within the interior space (104).

14. The intraoral interface structure according any of the preceding claims, comprising a sensor arrangement (200) comprising at least one sensor device attached to said frame member (102) for measuring at least one feature of said frame member (102) and/or a coupling medium (164) within the interior space (104).

15. The intraoral interface structure according claim 14, wherein the sensor arrangement (200) comprises a temperature sensor (208) for measuring a temperature of a coupling medium (164) within said interior space (104).

16. The intraoral interface structure according claim 14 or 15, wherein the sensor arrangement (200) comprises a force sensor (206) for measuring a force acting on the frame member (102).

17. The intraoral interface structure according any of the claims 14 to 16, wherein the sensor arrangement (200) comprises an accelerometer (204) for determining movement of the frame member (102).

18. A system for scanning a patients mouth comprising:
an intraoral scanning device (1) for recording ultrasound scan data in the mouth of the patient, the device comprising:
a housing (2) having a proximal portion (4) and a distal portion (6), the distal portion (6) being adapted for placement within the patients mouth;
a high frequency sonic head (20) comprising at least one transducer (36) for scanning a covered or non-covered tooth region (90) or residual tooth region (92) and mounted within the distal portion (6) of the housing (2), and
means for applying excitation signals to the sonic head (20) in order thereby to produce an ultrasonic wave which passes over at least part of the tooth region (90) or residual tooth region (92) such that the sonic head (20) receives ultrasonic waves reflected by the tooth region (90) or residual tooth region (92) to be scanned,
**characterized in that** the sonic head (20) is movable at least in a first and a second degree of freedom,
and by a drive assembly (22) arranged within the proximal portion (4) of the housing (2) for driving the sonic head (20) in the first and second degree of freedom such that the sonic head (20) is movable in a plane for applying the ultrasonic wave to a predetermined region of the tooth region or residual tooth region;
and an intraoral interface structure (100) according to any of claims 1 to 17.

19. A method for scanning a tooth region or a residual tooth region, comprising the steps:
- placing an intraoral interface structure (100) according to any of claims 1 to 17 in a patients mouth such that a tooth region (90) or a residual tooth region to be scanned is at least partially received in said interior space (104);
- filling the interior space (104) with a coupling medium (164) for impedance matching;
- engaging the receiving portion with a section of an intraoral scanning device (1); and
- scanning the tooth region (90) or a residual tooth region by emitting ultrasound waves to the tooth region (90) or a residual tooth region.
